# EUROPEAN PATENT APPLICATION

(11) **EP 1 849 780 A1**
(43) Date of publication of application: **31.10.2007**
(21) Application number: 06112873.2
(22) Date of filing: 21.04.2006
(51) Int. Cl.: C07D 401/04, A61K 31/465, A61K 39/385

(54) **Vaccine against nicotine addiction**

(71) Applicant: De Staat der Nederlanden, vert. door de minister van VWS, 2500 EJ The Hague (NL)
(72) Inventor: Hoogerhout, Peter, Bilthoven 3721 CN (NL); Zomer, Gijsbert, 3707 BE, Zeist (NL)
(74) Representative: van Westenbrugge, Andries

(57) **Abstract**

The present invention provides a hapten in the form of a novel nicotine derivative that may suitably be conjugated with an appropriate carrier to yield an effective vaccine against nicotine addiction. More particularly, the invention relates to a nicotine derivative of the following formula (I):

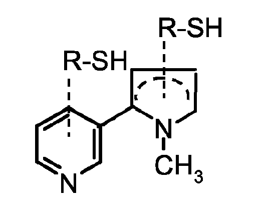

The present invention also relates to a hapten-carrier conjugate derived from the aforementioned nicotine derivative and a vaccine composition comprising said hapten-carrier conjugate.

## Description

### TECHNICAL FIELD OF THE INVENTION

The present invention provides a vaccine against nicotine addiction. More particularly, the present invention provides a hapten in the form of a novel nicotine derivative that may suitably be conjugated with an appropriate carrier to yield an effective vaccine against nicotine addiction.

### BACKGROUND OF THE INVENTION

According to the World Health Organisation, there are more than a billion smokers worldwide. It is estimated that about four million die each year because of cancer, heart disease and other smoking related illnesses.

It is widely accepted that people continue to smoke because tobacco contains nicotine, which is an addictive chemical. Many smoking cessation strategies, in fact, provide cigarette addicts with nicotine from sources other than tobacco, such as patches or gum.

An alternative cessation strategy is to provide a vaccine that stimulates the immune system to clear nicotine from the system by producing antibodies against nicotine. Thus, if following immunisation with a nicotine vaccine, an individual smokes a cigarette, the antibodies will clear the nicotine from the system before it reaches the brain. As a result, the expected stimulating effect of nicotine will not be experienced or be significantly reduced. Because the smoker will experience a lessening or cessation of the stimulating effect of nicotine, he/she will lose the desire to smoke.

Nicotine vaccines for use in smoking cessation strategies have been described in a number of patent publications:
WO 99/61054, for instance, describes a nicotine immunogen comprising a 5- or 6-nicotinyl-linker-carrier protein.
Furthermore, US 6,232,082 describes nicotine-carrier conjugates in which the carrier is bonded to the nicotine residue in the 3', 4' or 5'-position. The synthesis of these conjugates as described in the US patent is very complex. Furthermore, the immunogenicity studies described in the patent made use of complete and incomplete Freund's adjuvant to boost the immune response. This adjuvant, however, is not allowed for use in humans.
WO 2004/09116 describes a method of producing a nicotine-Qβ-conjugate comprising the steps of synthesising the N-hydroxysuccinimide ester ofO'-succinyltrans-3'-hydroxymethylnicotine and allowing the nicotine derivative to react with lysines on the surface of Qβ under formation of an amide bond. An important drawback of these conjugates resides in their poor *in vivo* stability.
WO 02/49667 describes a cotinine conjugate wherein the cotinine is conjugated to a carrier via the 1, 2, 5, 6 or 4'-position. In the international application it is observed that cotinine can antagonise the effects of nicotine *in vivo.* Furthermore, it is hypothesised that an immune reaction against cotinine can reduce the dampening effect of cotinine on the nicotine neurological effect, thereby resulting in the subject craving a lesser amount of nicotine. This hypothesis is not plausible, especially not since it is not substantiated by any experimental evidence.

### SUMMARY OF THE INVENTION

The present inventors have developed a novel hapten that can advantageously be used in the production of highly effective nicotine vaccines.

The hapten according to the present invention is a nicotine derivative of the following formula: 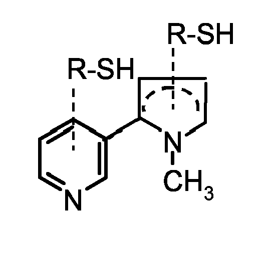 wherein either the five-membered ring or the six-membered ring is substituted with one R-SH radical, wherein R represents: Q-X₁-Y-Z-X₂:
- Q representing CH₂ or CO;
- X₁ representing a covalent bond, a linear or branched C₁-C₂₂ alkylene, a linear or branched C₂-C₂₂ alkenylene;
- Y representing O, S, NH, NH-CO, CO-NH, NH-NH-CO, NH-CO-NH, CO-NH-NH, CO-NH-NH-CO or S-S; and
- Z representing a covalent bond or CH(A), wherein A represents CO(OR₁), CH₂(OR₁), CH₂(NR₁R₂) or CH₂(OR₁), R₁ and R₂ independently representing hydrogen, a linear or branched C₁-C₄ alkyl or a linear or branched C₂-C4 alkenyl; and
- X₂ representing a linear or branched C₁-C₂₂ alkylene, a linear or branched C₂-C₂₂ alkenylene or a polyalkylene glycol moiety.

The aforementioned nicotine derivatives may be used to produce an immunogenic nicotine-carrier conjugate that can advantageously be used in the therapeutic or prophylactic treatment of nicotine addiction, said treatment comprising administration of the conjugate to an individual suffering from nicotine addiction or being at risk of becoming nicotine addicted. The nicotine-carrier conjugates of the present invention are highly stable and can be stored for several months or even years without significant loss of immunogenic efficacy. Also, *in vivo* stability of the present conjugates is very high, especially if the nicotine derivative is bound to the conjugate by a succinimide crosslinker and the nicotine derivative and crosslinker are linked by a thioether bond. Furthermore, the present nicotine-carrier conjugates can be produced using not more than a couple of relatively simple synthesis steps, yielding a well-defined conjugate product in high yield.

### DETAILED DESCRIPTION OF THE INVENTION

Accordingly, one aspect of the invention relates to a nicotine derivative of the following formula (I): 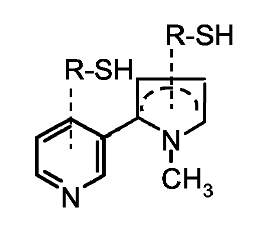 wherein either the five-membered ring or the six-membered ring is substituted with one R-SH radical, wherein R represents: Q-X₁-Y-Z-X₂:
- Q representing CH₂ or CO;
- X₁ representing a covalent bond, a linear or branched C₁-C₂₂ alkylene, a linear or branched C₂-C₂₂ alkenylene;
- Y representing O, S, NH, NH-CO, CO-NH, NH-NH-CO, NH-CO-NH, CO-NH-NH, CO-NH-NH-CO or S-S; and
- Z representing a covalent bond or CH(A), wherein A represents CO(OR₁), CH₂(OR₁), CH₂(NR₁R₂) or CH₂(OR₁), R₁ and R₂ independently representing hydrogen, a linear or branched C₁-C₄ alkyl or a linear or branched C₂-C₄ alkenyl; and
- X₂ representing a linear or branched C₁-C₂₂ alkylene, a linear or branched C₂-C₂₂ alkenylene or a polyalkylene glycol moiety.

In the above formula I, X₁ preferably represents a covalent bond or C₁-C₃ alkylene. Most preferably, X₁ represents a covalent bond.

According to another, particularly preferred embodiment, in formula I, Y represents NH or O. Most preferably, Y represents NH.

According to further preferred embodiment, Z in formula (I) represents a covalent bond. Alternatively, Z represents CH(A) and R₁ and R₂ independently represent hydrogen or C₁-C₂ alkyl.

In accordance with formula (I), X₂ may represent a polyalkylene glycol moiety. Preferably, said polyalkylene glycol moitey is a polyethylene glycol moiety or a polypropylene glycol moiety. According to a particularly preferred embodiment, X₂ in formula (I) represents a C₁-C₄ alkylene, preferably C₁-C₃ alkylene, especially ethylene.

The R-SH residue in formula (I) may be bonded to the 3', 4' or 5' position of the five-membered ring of the nicotine molecule. Preferably, R is bonded to the 3'- or 4'- position, most preferably to the 3'-position of said five-membered ring.

According to another particularly preferred embodiment, the nicotine derivative of formula (I) is trans-substituted with the R-SH residue. In another particularly preferred embodiment, the nicotine derivative of the present invention is a L-nicotine derivative.

Preferred nicotine derivatives according to the present invention include:
- 3'-nicotine-N-(2-mercaptoethyl)carboxamide
- 3'-(2-mercaptoethylaminomethyl)-nicotine,
- N-(3'-nicotinylcarbonyl)cysteinol,
- N-(3'-nicotinylmethyl)cysteinol,
- N^{α}-(3'-nicotinylcarbonyl)cysteine amide,
- N^{α}-(3'-nicotinylmethyl)cysteine amide,
- 3'-nicotine-N-[(1-aminomethyl-2-mercapto)ethyl]carboxamide,
- 3'-[(1-aminomethyl-2-mercapto)ethylaminomethyl]nicotine

Especially preferred nicotine derivatives of the present invention are 3'-nicotine-N-(2-mercaptoethyl)carboxamide and 3'-(2-mercaptoethylaminomethyl)-nicotine.

Another aspect of the present invention relates to a hapten-carrier conjugate of the following formula (II): 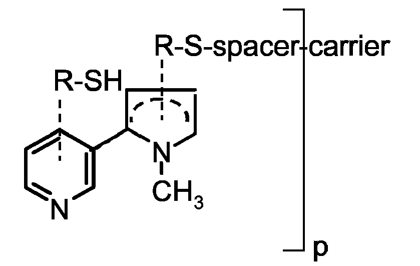 wherein either the five-membered ring or the six-membered ring is substituted with one R-SH radical; p is 1 to 500; and R has the same meaning as defined herein before in relation to formula (I).

The carrier employed in the hapten-carrier conjugate of the present invention may be any immunogenic material that can safely be used in humans and that can be linked chemically to the present nicotine derivative. In a preferred embodiment, the carrier is selected from the group consisting of immunogenic substances, viruses, virus-like particles, protein complexes, proteins, polypeptides, liposomes and immuno-stimulating complexes (ISCOM). According to a particularly preferred embodiment, the carrier is an immunogenic protein or polypeptide. Particularly suitable examples of immunogenic proteins include tetanus toxoid, diphtheria toxoid, keyhole limpet hemocyanin (KLH), hemocyanine, albumine, non-toxic mutant diphtheria toxoid CRM197, outer membrane protein complex (OMPC) from Neisseria meningitidis, the B subunit of heat-labile Escherichia coli, recombinant exoprotein A from Pseudomonas aeruginosa (rEPA) and a virus-like particle assembled from recombinant coat protein of bacteriophage Qb.

The spacer in the present immunoconjugate covalently links the nicotine derivate to the carrier. Spacers that can be used to crosslink haptens and carriers (especially protein/polypeptide components) are well-known in the art. Preferably, in the present invention, the spacer is a residue of a heterobifunctional crosslinker, notably a succinimidyl crosslinker, like:
- N-succinimidyl bromoacetate,
- N-succinimidyl 3-(bromoacetamido)propionate (SBAP),
- N-(ε-maleimidocaproyloxy)succinimide ester (EMCS),
- N-(N-maleimidylpropionyl)-9-amino-4,7-dioxanonaoic acid N-succinimide ester (NHS-PEG2-maleimide), or
- N-succinimidyl 3-(2-pyridyldithio)propionate (SPDP),
which crosslinker is used to modify the carrier to give a sulfhydryl-reactive carrier. In a second step, the nicotine derivative is coupled to the sulfhydryl-reactive protein to give the immunoconjugate wherein the spacer derived from the heterobifunctional crosslinker connects the nicotine derivative to the carrier. According to a preferred embodiment, in the present conjugate, the hapten and spacer are linked together by a thioether bond. Conjugates in which the hapten and spacer are bound by a thioether bond offer the advantage of high *in vivo* stability.

A further aspect of the present invention relates to the use of a hapten-carrier conjugate as defined herein before in the preparation of a vaccine composition, especially a vaccine composition for use in a method of preventing or treating nicotine addiction, said method comprising administering a therapeutically effective amount of the hapten-carrier conjugate. The conjugates of the present invention offer the advantage that they are highly stable under *in vivo* conditions. Furthermore, the present hapten-carrier conjugates can be prepared in a well-defined form which means that the risk of undesired side-effects is effectively minimised.

Suitable modes of administration include subcutaneous, intramuscular, transmucosal and intravenous administration. Most preferably, the vaccine composition is administered subcutaneously or intramuscularly.

The addictive effect of nicotine is believed to be associated with the capability of nicotine to cross the blood-brain barrier. The present method of treating or preventing nicotine addiction relies on preventing nicotine from crossing the blood brain barrier. In particular, administration of a nicotine hapten-carrier conjugate to a person will generate antibodies against nicotine, in the bloodstream of the person. If the person smokes, the nicotine in his blood will be bound by the circulating anti-nicotine antibodies, preventing the nicotine from reaching the brain. Therefore, the antibodies will prevent the physiological and psychological effects of nicotine that originate in the brain. Because the smoker will experience a lessening or cessation of these effects, he/she will lose the desire to smoke. The same therapeutic effects are expected if a person uses smokeless tobacco, after being immunized with a nicotine hapten-carrier conjugate of the invention. Additionally, the conjugates and antibodies of the invention may exert their effects by affecting the ability of nicotine to stimulate the peripheral nervous system.

The conjugates of the invention are suitable for treating and preventing nicotine addiction. For treating nicotine addiction, the nicotine-carrier conjugate of the invention is administered to a person suffering from nicotine addiction. For preventing nicotine addiction, persons at risk for developing nicotine addiction are treated with the conjugate according to the invention.

A vaccine composition of the present invention comprises at least one nicotine hapten-carrier conjugate in an amount sufficient to elicit an immune response thereto. Initial vaccination with the nicotine hapten carrier conjugate of the present invention creates high titers of antibodies that are specific to nicotine. The therapeutically effective amount of a conjugate which is administered to a person in need of treatment for nicotine addiction is readily determined by the skilled artisan. Suitable dosage ranges are 1-1000 µg/dose. It generally takes a person one to several weeks to generate antibodies against a foreign antigen. The production of antibodies in a person's blood can be monitored by using techniques that are well-known to the skilled artisan, such as ELISA, radioimmunoassay, surface plasma resonance, and Western blotting methods. Therapeutic effectiveness also can be monitored by assessing various physical effects of nicotine, such as blood pressure.

It will be understood that initial administration of the present immunogenic conjugate may be followed by subsequent administration of one or more "boosters" of conjugate. Such a booster will increase the production of antibodies against the nicotine hapten-carrier conjugate of the invention.

The vaccine compositions of the present invention may contain at least one adjuvant. The adjuvant used in the present invention will be selected so that the effect of the carrier protein is not inhibited. Adjuvants used in the present invention are those which are physiologically acceptable to humans, these include, but are not limited to aluminium hydroxide, aluminium phosphate, oil/surfactant based emulsion adjuvants such as Montanide ™ in which different surfactants (especially mannityl oleate) are combined with a mineral oil, squalene-containing emulsions such as MF59™, monophosphoryl lipid A, or Neisseriae mutant lipopolysaccharide (as described in PCT/NL98/0063).

The vaccine compositions of the present invention may optionally contain one or more pharmaceutically acceptable excipients. Suitable excipients include sterile water, salt solutions and buffers. According to a preferred embodiment, the vaccine composition the present hapten-carrier conjugate solubilised in an aqueous, saline solution at a pharmaceutically acceptable pH. Alternatively, the vaccine composition comprises a suspension of the hapten-carrier conjugate.

Additionally, the vaccine composition may optionally contain at least one auxiliary agent, such as dispersion media, coatings, microspheres, liposomes, microcapsules, lipids, surfactants, lubricants, preservatives and stabilizers.

The vaccine composition of the present invention preferably is sterile. Furthermore, the composition may contain components that preserve against infestation with, and growth of, micro-organisms.

It is preferred that the vaccine composition is manufactured in the form of a sterile aqueous liquid which is ready for immediate administration.

The nicotine derivates according to the present invention can advantageously be linked to carriers using synthesis routes that do not produce protein-protein conjugates. Because such protein-protein conjugates adversely affect the solubility as well as the efficacy of the vaccine composition, another aspect of the invention relates to a vaccine composition comprising a hapten-carrier conjugate according to the present invention and a pharmaceutically acceptable excipient, wherein the vaccin composition contains no protein-protein conjugates.

The invention is further illustrated by means of the following examples.

### EXAMPLES

### Example 1

### Trans-3'-nicotine-N-(2-mercaptoethyl)carboxamide (3) was synthesised as follows:

### N,N'-Bis-(trans-4'-cotininylcarbonyl)cystamine (1)

A mixture of 1.00 g (4.54 mmol) *trans*-4'-cotininecarboxylic acid, 0.45 g (2.00 mmol) cystamine dihydrochloride and 0.68 g (4.44 mmol) *N*-hydroxybenzotriazole was concentrated from 25 ml dry *N,N-*dimethylformamide twice. The residue was dissolved in 25 ml dry *N,N-*dimethylformamide and 0.80 ml (4.59 mmol) *N,N-*diisopropylethylamine and 0.68 ml (4.39 mmol) *N,N'*-diisopropylcarbodiimide were added. The mixture was stirred at room temperature. After 20 h, 4 ml (0.2 mol) water was added and stirring was continued for 1 h. Then, the mixture was concentrated *in vacuo.* The residue was taken up in 100 ml 0.5 M KHSO₄ and washed with chloroform (3 × 25 ml). Solid Na₂CO₃.H₂O (8.0 g) was added slowly to the aqueous layer which was then extracted with chloroform (3 × 25 ml). The organic layers were combined, dried (MgSO4), filtered and concentrated to give compound 1 as a syrup. Ion-spray mass spectrometry: MH⁺ (found/calculated) 557/557.

The trifluoroacetic acid salt of compound 1 was prepared by adding trifluoroacetic acid (0.89 ml, 12 mmol) to the filtrate which was then concentrated. The residue was concentrated from methanol (2 × 10 ml); ¹H NMR (D₂O), δ 8.84-8.86 (m, 2x2H), 8.59-8.64 (m, 2×1H), 8.17 (dd, 2×1H), 5.14 (d, 2×1H), 3.45-3.85 (m, 2x2H), 3.17-3.24 (m, 2×1H), 2.93-3.02 (m, 2×1H), 2.76-2.86 (m, 2x3H), 2.71 (s, 2×3H). ¹³C NMR (D₂O), δ 180.0, 176.4, 149.2, 145.5, 143.9, 142.9, 131.8, 68.0, 50.1, 41.6, 40.1, 37.5, 32.0 (signals of trifluoroacetate were unresolved). The syrup obtained was used in the next step without purification.

### Trans-4'-cotinine-N-(2-mercaptoethyl)carboxamide (2)

Compound **1** (≈ 2 mmol, free base) was dissolved in 20 ml methanol and concentrated. The residue was redissolved in 20 ml methanol / water, 99/1, v/v, and 0.60 g (2.10 mmol) *tris*-(2-carboxyethyl)phosphine hydrochloride was added. The mixture was stirred at room temperature for 3 h, in which period the phosphine gradually dissolved. Then, the mixture was diluted with 100 ml chloroform and washed with 50 ml 1 M NaHCO₃. The aqueous layer was extracted with chloroform (2 × 25 ml). The combined organic layers were washed with 50 ml 1 M NaHCO₃ once more and again the aqueous layer was extracted with chloroform (2 × 25 ml). The organic layers were combined, dried (MgSO₄), and filtered. The filtrate was concentrated to give 2 as a syrup of approximately 97% purity, as determined by TLC and HPLC.

HPLC-analysis was carried out using a Waters Alliance 2695 HPLC which was equipped and operated as follows:
Column: C18, 2.1x150 mm, 3 µm (Waters BioSuite PA-A).
Flow: 0.2 ml/min, splitter na UV detector
Eluens A: Water, 0.1 % TFA
Eluens B: Acetonitrile, 0.08 % TFA
Gradient: in 20 min from 5% to 67% eluens B
Detection: UV - Waters PDA 996: 254 nm
   Mass - Micromass Quattro Micro API: ES+

Ion-spray mass spectrometry: MH⁺ (found/calculated) 280/280. ¹H NMR (CDCl₃), δ 8.61(dd, 1H), 8.52 (d, 1H), 7.58 (dt, 1H), 7.34-7.39 (m, 1H), 6.04 (br, 1H), 4.77 (dd, 1H), 3.33-3.50 (m, 2H), 2.72-2.88 (m, 3H), 2.72-2.88 (m, 2H), 2.65 (s, 3H).1.26 (t, 1H). ¹³C NMR (CDCl₃), δ 173.2, 170.8, 150.2, 148.7, 135.3, 134.6, 124.3, 65.0, 48.6, 42.6, 34.6, 28.6, 24.6.

### Trans-3'-nicotine-N-(2-mercaptoethyl)carboxamide (3)

Crude compound **2** (≈ 4 mmol) was concentrated from dry tetrahydrofuran (2 × 10 ml) and dissolved in 20 ml dry tetrahydrofuran. The solution was stirred under nitrogen at room temperature and 2.0 ml of 2 M LiAlH₄ in tetrahydrofuran was added dropwise. Stirring at room temperature was continued for 2 h. The solution was cooled in an ice-bath and 300 µl (16.7 mmol) water in 2.7 ml tetrahydrofuran was added dropwise, followed by 20 ml 1 *N* HCl. Then, 100 ml 1 M NaHCO₃ was added slowly and the mixture was extracted with chloroform (3 × 25 ml). The organic layers were combined, dried (MgSO₄) and filtered. Trifluoroacetic acid (0.89 ml, 12 mmol) was added to the filtrate which was then concentrated. The residue was concentrated from methanol (2 × 10 ml).

HPLC/MS (methodology described above) showed that **3** (MH⁺=266) was the major product. A significant side-product showed a molecular ion at 264 amu, whereas *trans*-3'-(2-mercaptoethylaminomethyl)nicotine (**4,** MH⁺=252) was a minor side-product. Compound **3** was purified from the mixture by preparative HPLC.

HPLC-purification was carried out using a Waters 600E which was equipped and operated as follows:
Column: C18, 2.1x150 mm, 5 µm, 300 Angstrom (Vydac 218TP510)
Flow: 4.5 ml/min
Eluens A: Water, 0.1 % TFA
Eluens B: Acetonitrile, 0.08 % TFA
Gradient: in 20 min from 5% to 67% eluens B
Detection: UV - Waters 486: 254 nm
Compound **3** (free base) - ¹H NMR (CDCl₃): δ 8.48-8.58 (m, 2H), 7.72-7.77 (m, 1H)7.24-7.30 (m, 1H), 5.95 (br, 1H), 3.21-3.47 (m, 4H), 2.50-2.74 (m, 4H), 2.12-2.26 (m + s, 5H), 1.18 (t, 1H); ¹³C NMR (CDCl₃): δ 173.2, 149.2, 149.0, 136.14, 136.12, 124.1, 72.5, 56.1, 54.6, 42.3, 39.8, 27.3, 24.5.

### Example 2

### Trans-3'-(2-mercaptoethylaminomethyl)nicotine (4) was synthesised as follows:

Crude compound **2** (≈ 2 mmol) as described in example 1 was concentrated from dry tetrahydrofuran (2 × 5 ml) and dissolved in 10 ml dry tetrahydrofuran. The solution was added slowly to 10 ml 1M borane-tetrahydrofuran complex in tetrahydrofuran under a nitrogen atmosphere. The reaction mixture was stirred at room temperature for 20 h. Dry methanol (5 ml) was added and the mixture was concentrated. The residue was concentrated from dry methanol (3 × 10 ml) and dissolved in 10 ml 1 N HCl. Then, 50 ml 1 M NaHCO₃ was added slowly and the mixture was extracted with chloroform (3 × 25 ml). The organic layers were combined, dried (MgSO₄) and filtered. Trifluoroacetic acid (0.45 ml, 6 mmol) was added to the filtrate which was then concentrated. The residue was concentrated from methanol (2 × 10 ml).

### Example 3

Immunoconjugates are prepared starting from the thiol-containing haptens described in Examples 1 and 2, using the methodology described by J.W. Drijfhout and P. Hoogerhout: "Methods of preparing peptide-carrier conjugates" In: Fmoc Solid Phase Peptide Synthesis: A Practical Approach (W.C. Chan and P.D. White, eds.). Oxford University Press, 2000, pp. 229-241.

### Materials

- Carriers: tetanus toxoid (TTd), keyhole limpet hemocyanin (KLH), or bovine serum albumin, (BSA).
- Cross-linkers: N-succinimidyl bromoacetate, N-succinimidyl 3-(bromoacetamido)propionate (SBAP), N-(N-maleimidylpropionyl)-9-amino-4,7-dioxanonaoic acid N-succinimide ester (NHS-PEG2-maleimide), and N-succinimidyl 3-(2-pyridyldithio)propionate (SPDP).

The carrier is dissolved in 0.1 M sodium phosphate buffer, pH 8, at a concentration of 3.0 mg/ml. The cross-linker is dissolved freshly in *N,N*-dimethylacetamide at a concentration of 80 mM. An aliquot of 50 µl of the solution of the cross-linker (i.e. 4 µmol cross-linker) is added to 1.75 ml of the carrier solution. The solutions are mixed and left to stand at room temperature for 1 h. Then, the reaction mixture is applied to a PD-10™ column equilibrated in 0.1 M sodium phosphate buffer, containing 5 mM EDTA, pH 6 (de-aerated with helium). Elution is effected with the same buffer. The modified protein is collected in a volume of 3.0 ml and used immediately for conjugation of the thiol-containing haptens.

The purified thiol-containing hapten (trifluoroacetate) (3-4 µmol) is dissolved in 250 µl water and added to 2.0 ml of a freshly prepared solution of the sulfhydryl-reactive carrier in 0.1 M sodium phosphate buffer, containing 5 mM EDTA, pH 6. The solutions are mixed and left to stand at room temperature for 16-24 h. Then, the reaction mixture is applied to a PD-10™ column equilibrated in phosphate buffered saline, pH 7.2 (PBS). Elution is effected with the same buffer. The immunoconjugate (≈ 3.5 mg) is collected in a volume of 3.5 ml and stored at 2-8 °C until the preparation of a vaccine.

### Example 4

### Vaccines and immunization

Vaccines are prepared by appropriate dilution of the stock solutions (1 mg/ml) of the conjugates prepared from the compounds 3 and 4 and bromoacetylated TTd, bromoacetylated KLH, (bromoacetamido)propionylated TTd, and (bromoacetamido)propionylated KLH with a suspension of aluminium phosphate (0.5-1.5 mg/ml) in PBS, pH 7. Plain PBS is diluted with aluminium hydroxide suspension for obtaining a mock vaccine. Groups of eight mice are immunized on days 0, 14, and 28 with 0.5 ml of vaccine containing 0.1-100 µg of immunoconjugate. Sera are taken on days 0, 28 and 42 and stored at -20 °C until use.

### Example 5

### Antibody titers

Antibody titers in the sera of Example 4 are determined by inhibition enzyme-linked immunosorbent assay (inhibition ELISA). The hapten-BSA conjugates of Example 3 are used as coating antigens. Nicotine, cotinine, or acetylcholine were used as inhibitors.

The conjugates tested are found to induce satisfactory antibody titers in mice. The response is nicotine-specific. The antibodies do not cross-react significantly with cotinine or acetylcholine.

## Claims

1. A nicotine derivative of the following formula: 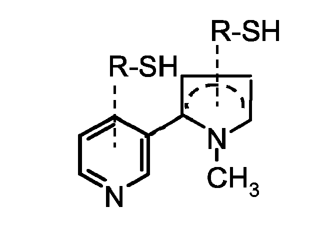 wherein either the five-membered ring or the six-membered ring is substituted with one R-SH radical, wherein R represents: Q-X₁-Y-Z-X₂:
- Q representing CH₂ or CO;
- X₁ representing a covalent bond, a linear or branched C₁-C₂₂ alkylene, a linear or branched C₂-C₂₂ alkenylene;
- Y representing O, S, NH, NH-CO, CO-NH, NH-NH-CO, NH-CO-NH, CO-NH-NH, CO-NH-NH-CO or S-S; and
- Z representing a covalent bond or CH(A), wherein A represents CO(OR₁), CH₂(OR₁), CH₂(NR₁R₂) or CH₂(OR₁), R₁ and R₂ independently representing hydrogen, a linear or branched C₁-C₄ alkyl or a linear or branched C₂-C₄ alkenyl; and
- X₂ representing a linear or branched C₁-C₂₂ alkylene, a linear or branched C₂-C₂₂ alkenylene or a polyalkylene glycol moiety.

2. Nicotine derivative according to claim 1, wherein X₁ represents a covalent bond or C₁-C₃ alkylene, preferably a covalent bond.

3. Nicotine derivative according to any one of claim 1 or 2, wherein Y represents NH or O, preferably NH.

4. Nicotine derivative according to any one of the preceding claims, wherein Z represents a covalent bond.

5. Nicotine derivative according to any one of claims, wherein Z represents CH(A) and R₁ and R₂ independently represent hydrogen or C₁-C₂ alkyl.

6. Nicotine derivative according to any one of the preceding claims, wherein X₂ represents a C₁-C₄ alkylene, preferably ethylene.

7. Nicotine derivative according to any one of the preceding claims, wherein R is bonded to the 3'- or 4'- position, preferably to the 3'-position of the five-membered ring.

8. Nicotine derivative according to any one of the preceding claims, wherein the derivative is 3'-nicotine-N-(2-mercaptoethyl)carboxamide

9. Nicotine derivative according to any one of the preceding claims, wherein the derivative is 3'-(2-mercaptoethylaminomethyl)-nicotine

10. A hapten-carrier conjugate of the following formula: 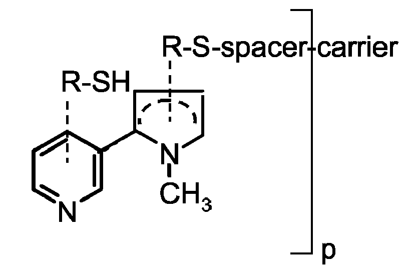 wherein either the five-membered ring or the six-membered ring is substituted with one R-SH radical; p is 1 to 500; and R has the same meaning as in claim 1.

11. The hapten-carrier conjugate according to claim 10, wherein the carrier is selected from the group consisting of immunogenic substances, viruses, virus-like particles, protein complexes, proteins, polypeptides, liposomes and immuno-stimulating complexes (ISCOM).

12. The hapten-carrier conjugate according to claim 11, wherein the carrier is a protein selected from the group consisting of tetanus toxoid, diphtheria toxoid, keyhole limpet hemocyanin (KLH), hemocyanine, albumine, non-toxic mutant diphtheria toxoid CRM197, outer membrane protein complex (OMPC) from Neisseria meningitidis, the B subunit of heat-labile Escherichia coli, recombinant exoprotein A from Pseudomonas aeruginosa (rEPA) and a virus-like particle assembled from recombinant coat protein of bacteriophage Qb.

13. Use of a hapten-carrier conjugate in the preparation of a vaccin composition for use in a method of preventing or treating nicotine addiction, said method comprising administering a therapeutically effective amount of the hapten-carrier conjugate according to any one of claims 10-12.

14. A vaccine composition comprising the hapten-carrier conjugate according to any one of claims 10-12 and a pharmaceutically acceptable excipient, wherein the vaccin contains no protein-protein conjugates.
